# EUROPEAN PATENT SPECIFICATION

(11) **EP 2 290 048 B1**
(45) Date of publication and mention of the grant of the patent: **29.02.2012**
(21) Application number: 09011042.0
(22) Date of filing: 28.08.2009
(51) Int. Cl.: C12M 1/00

(54) **Photo-bioreactor and building having a photo-bioreactor mounted on a wall thereon**
Photobioreaktor und Gebäude mit Photobioreaktor an einer Wand davon
Photo-bioréacteur et bâtiment doté d'un photo-bioréacteur monté sur un de ses murs

(43) Date of publication of application: 02.03.2011
(73) Proprietor: King Abdulaziz City for Science and Technology, Riyadh 11442 (SA)
(72) Inventor: Baabbad, Mazen, 11442 Riyadh (SA)
(74) Representative: Goddar, Heinz J.

(56) References cited:
- EP-A- 0 889 118
- JP-A- 10 080 267
- JP-A- 2000 320 041
- KR-A- 20050 013 269
- US-A1- 2009 029 445

## Description

The present invention relates to a photo-bioreactor for growing algae and to a building having such a photo-bioreactor mounted on a wall or surface thereon.

Algae cultivation has received increased attention in the past decades due to their high nutrients content and their ability to form biomass with much less water than any known land plant. Algae can have an oil content up to 40% of their dry weight, which can be used to produce bio-diesel. Algae culture can be grown in a number of different types of incubators, which are called photo-bioreactors, with each type having its advantages and disadvantages and differences in cost and efficiency. Also, photo-bioreactors differ in their susceptibility and safety against irreversible biological and chemical contamination. Photo-bioreactors for growing algae have to provide suitable amounts of light, nutrients, temperature, chemical composition as well as steering motion and oxygen removal to the algae culture.

In the prior art, four major types of photo-bioreactors are known: open pounds, vertical column photo-bioreactors, flat-plate photo-bioreactors and tubular photo-bioreactors, see O. Pulz, "Photobioreactors: production systems for phototrophic microorganisms", Applied Microbiol Biotechnol (2001) 57:287-293; and C.U. Ugwu, H. Aoyagi, H, Uchiyama, "Photobioreactors for mass cultivation of algae" Bioresource Technology 99 (2008) 4021-4028. Other types include plastic bags or a vertical wall like tubular arrangements. These designs were used to produce high value algae, for example with regard to nutrients content, pharmaceutical purposes, pigments, etc., but mostly not for fuel purposes because of the high cost, see F. Lehr and C. Posten, "Closed photo-bioreactors as tools for biofuel production" Current Opinion in Biotechnology 2009, 20:280-285.

However, some technologies are aiming at producing bio-fuels. A review of earlier designs, such as in US 3,986,297, shows a photo-bioreactor in the form of a tank with a central agitator and a plurality of nozzles to inject gas into the tank. The tank uses sources of light that are immersed in the tank and an outer tank for temperature control. However, this design appeared to not be economical since it does not use the naturally available light in addition to the difficulty in controlling the temperature when the system needs cooling during the summer time.

Other designs, such as disclosed in US 4,676,956, show a vertical tubular photo-bioreactor that is lighted from the inside by light source and from the outside. Gasses are fed in the reactor from its bottom. However, this system is rather complex and difficult to maintain.

KR-A-20050013269 and EP-A-0889118 describe photo-bioreactors comprising transparent containers and stirring means to harvest algae.

It is an object of the present invention to provide a photo-bioreactor for growing algae which overcomes the difficulties and disadvantages of the prior art. Especially, a photo-bioreactor shall be provided which has a simple design and can be easily assembled and integrated into or onto walls of a building, preferably a glass walled building, to especially share the air conditioning system with the building. The reactor shall be easily provided with nutrients and electricity.

This object is achieved by a photo-bioreactor for growing algae comprising: at least one transparent container having an at least partly round perimeter and containing algae culture, optional means for mounting the container on a vertical surface, preferably of a glass walled building, at least one feed gas tube, at least one gas removal hole, at least one feed liquid tube, at least one liquid discharge tube, preferably provided on the perimeter of the container, and rotatable stirring and removal means inside the container, wherein the stirring and removal means comprises a rotatable belt having protusions extending outwardly, and the belt being concavely deformed between the protusions, as well as a screw to be lowered into a concave deformation. Stirring and removal means in the prior art comprises at least one paddle.

The size of the paddle allows removal of the algae formed on inner walls of the container. In this regard, the size of the paddle is to be understood in that the lateral edges of the paddle are so close to the inner walls of the container that algae formed thereon can be removed by rotation of the stirring and removal means. This may result in a wiping effect of the paddle. In other words, the paddle allows scraping of the inner walls of the container to remove algae formed thereon.

According to the invention, the stirring and removal means comprises a rotatable belt having protrusions extending outwardly, and the belt being concavely deformed between the protrusions.

Preferably, the size of the protrusions allows removal of the algae formed on inner walls of the container. As for the paddle in the first embodiment, the size of the protrusions is to be understood in that the top edges of the protrusions are designed in that they are so close to the inner walls of the container to remove algae formed thereon. This may also result in a wiping effect of the protrusions. The material of the belt is preferably porous to allow ingrowth of algae. The material may be for example woven nylon.

The stirring and removal means comprises a screw adapted to be lowered into a concave deformation of the belt and to be raised therefrom. When the screw is rotated, it scrapes the algae from the belt's surface, and subsequently, the algae can be removed to the outside of the container.

It is additionally preferred that the photo-bioreactor comprises at least one drive mechanism for rotating the stirring and removal means.

In one embodiment, the stirring and removal means preferably comprises a bearing which is located above the level of liquids in the container.

In a further embodiment, the stirring and removal means preferably comprises a rotating shaft isolated from any contact with the liquids in the container.

The container may be a cylindrical disc container, or the container may have an elongated shape with rounded corners.

According to the invention is also provided a building, preferably an at least partly glass walled building, having at least one photo-bioreactor according to the invention mounted on a vertical surface thereof.

Preferred is a building, wherein the at least one photo-bioreactor is connected with the air conditioning system of the building, preferably the at least one feed gas tube of the reactor.

Surprisingly, it was found that the inventive photo-bioreactor can be easily integrated into or onto walls of a building, preferably a glass walled building. Such an integration provides significant cost cutting and also an innovative way to share the air conditioning system with that building. Especially discharged air from the air conditioning system comprising a higher concentration of carbon dioxide may be introduced into the inventive photo-bioreactor. Also, the piping system of the inventive photo-bioreactor can be easily provided in a building to which the photo-bioreactor is mounted. The photo-bioreactor may receive the necessary light for growing the algae from the inside of the building or the outside, i.e. sunlight.

The photo-bioreactor according to the present invention can be easily provided in a modular system and can be easily maintained. The modules can be rapidly isolated in the case of chemical or biological contamination. The design of the inventive photo-bioreactor uses simple components resulting in cost and maintenance reductions.

It is an essential feature of the inventive photo-bioreactor that no internal light source in the transparent container is necessary.

Additional features and advantages of the present invention will become, aware from the following detailed description of preferred embodiments on the basis of the drawings, wherein only figures 6-10 correspond to embodiments of the invention, in which
Figure 1 shows a photo-bioreactor;
Figure 2 shows the internal design of the photo-bioreactor of figure 1;
Figure 3 shows a paddle bearing of the photo-bioreactor;
Figure 4 shows a container central core of the reactor;
Figure 5 shows a photo-bioreactor in a modular design;
Figure 6 shows a photo-bioreactor according to the present invention;
Figure 7 shows the internal design of the photo-bioreactor of figure 6;
Figure 8 shows a more detailed view of the internal design of the photo-bioreactor of figure 7;
Figure 9 shows a more detailed view of the external design of the photo-bioreactor of figure 7; and
Figure 10 shows the photo-bioreactor according to the second embodiment in a modular design.

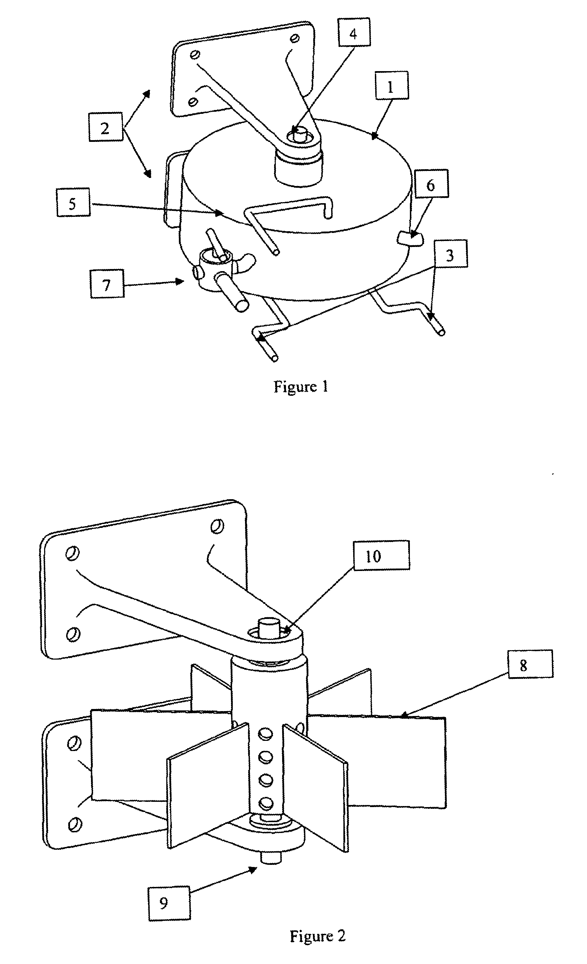

Figure 1 shows a photo-bioreactor. The photo-bioreactor comprises a cylindrical disc container 1 made from transparent material and containing algae culture (not shown). Preferably, the material of the container 1 is transparent plastic. The container 1 comprises means 2 for mounting the container 1 on a vertical surface, for example a vertical surface of a building, preferably a glass walled building. Two feed gas tubes 3 are provided at the bottom of the container 1 to allow introduction of algae feed gas into the container 1. Algae feed gas may be any gas comprising at least partly carbon dioxide, for example discharge gas from an air-conditioning system. At the top of the container 1 a hole 4 for discharging gases from the container is provided.

Also at the top of the container a feed liquid tube 5 is provided to allow introduction of nutrients, water, algae culture, etc.. At the perimeter of the cylindrical disc container 1 are provided two discharge tubes 6. All tubes 3, 5 and 6 may be provided with closing means, such as a three way valve 7 as shown at one discharge tube 6.

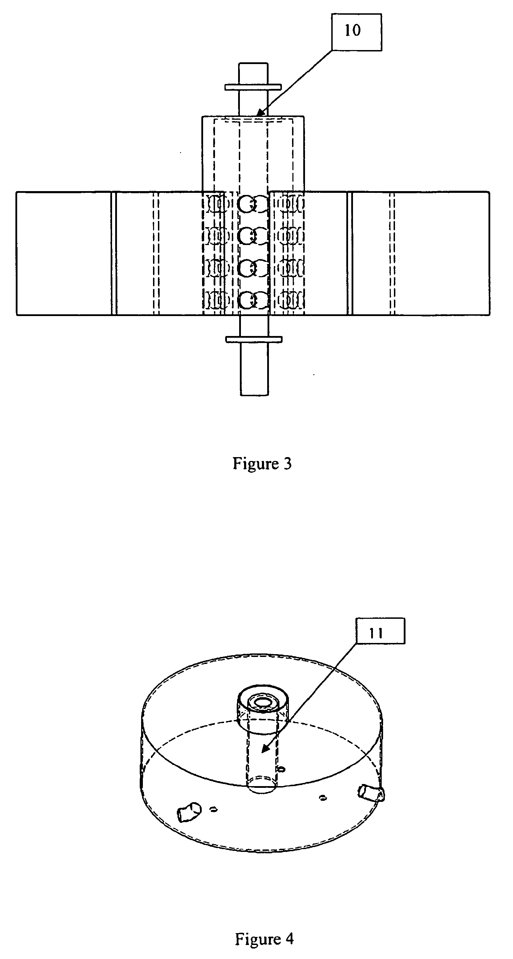

Figure 2 shows the internal design of the photo-bioreactor illustrated in figure 1. Inside the container 1 there is provided a paddle 8 with several blades, the size of individual blades is such that removal of algae formed on the inner walls of the container 1 is effected when the paddle is rotated. Rotation of the paddle 8 is effected by a motor (not shown), wherein the paddle 8 is provided with a rotating shaft 9 and bearing 10. As can be taken especially from figure 3, the paddle bearing 10 is located above the level of the liquids inside the container so as to avoid contamination of the algae with bearing lubricating oil. Additionally, the container 1 has a central core that allows the paddle rotating shaft 9 through the unit without getting into contact with the algae, see figure 4.

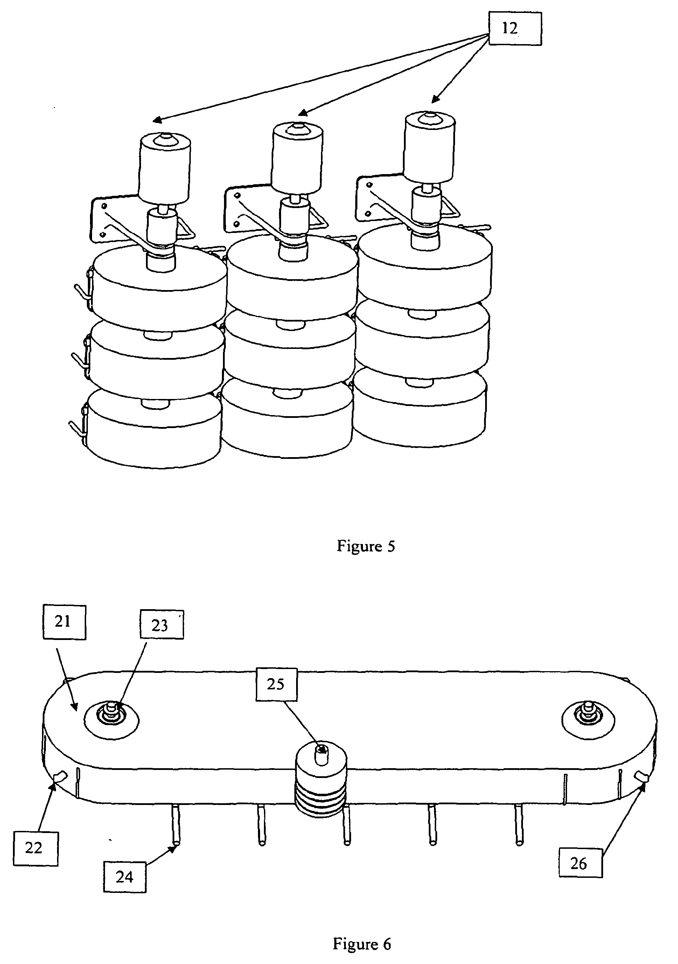

Further, figure 5 illustrates a modular design of a photo-bioreactor. Three containers are arranged on top of each other, three modular designs side by side, wherein each modular set up is driven by an electrical motor 12. The containers may be operated in series or parallel.

In operation, the container 1 is filled with or already contains algae culture. Algae feed gas which contains carbon dioxide, is fed to the container through the feed gas tubes 3. For example, the feed gas may be the discharge gas from an air conditioning system of a building. The gas introduced into the container 1 is allowed to escape through the upper hole 4 in the center of the container 1. Algae feed liquid which comprises water and nutrients is fed into the container by the feed liquid tube 5. If the container 1 is filled with algae culture and algae grow on the inner surfaces of the container 1, the paddle 8 is rotated around the center of the disc periodically to stir the algae culture and to remove the algae layer(s) that is (are) formed on the inner walls of the container 1. The removed algae are then discharged by the two discharge holes 6 provided at the perimeter of the container 1. In this regard, removal is preferably effected by rotation of the paddle 8 which creates a centrifugal force that, when the three way valve 7 is open, discharges algae out of the system and purges the system in case of biological or chemical contamination.

As especially demonstrated in figure 5, a number of photo-bioreactors can be mounted on building walls and share the air conditioning and day and night lightning, in addition to the possibility of obtaining the carbon dioxide from the building air. Further, a photo-bioreactor of the invention may supply oxygen into the building.

The inventive photo-bioreactor is illustrated in figures 6-10.

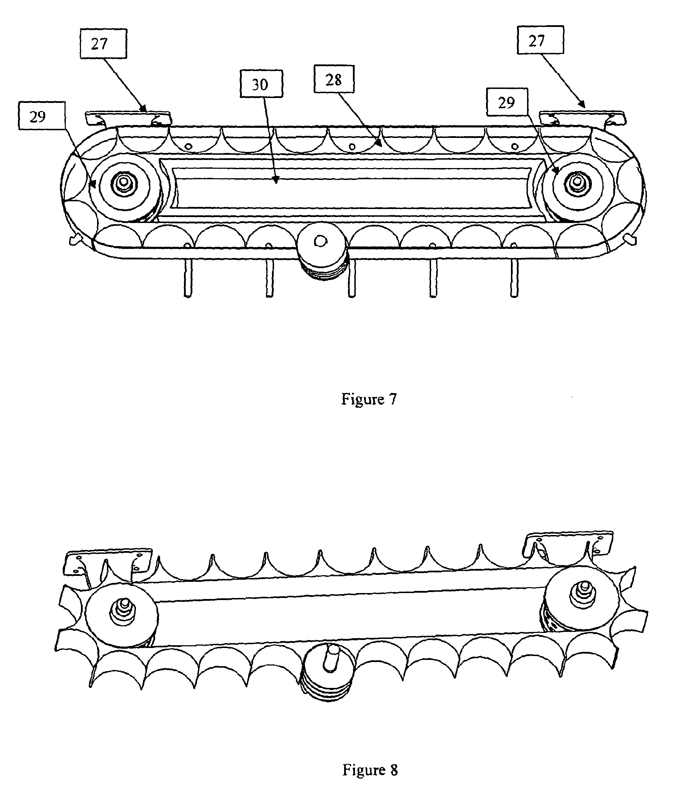

According to figure 6, the photo-bioreactor of the invention comprises a transparent container 21 which is in an elongated shape with rounded corners. Algae feed gas tubes 24 are provided at the bottom of the container 21, whereas excessive gas can escape via a hole (not shown) in the top of the container 21. Algae feed liquid (containing water and nutrients) can be introduced to the container 21 via feed liquid tube 22. Rotating shafts 23 are also provided which allow rotation of a belt 28 (see figure 7). Via discharge tube 26 algae may be removed. The container 21 of the second embodiment also comprises a screw 25 which is adapted to be lowered and raised, as will be explained in a greater detail below.

The internal design of the photo-bioreactor of the invention is disclosed in figure 7. Inside the container 21 a drivable belt 28 is provided, which can be driven by rotating belt pulleys 29. The container 21 as such may be mounted by respective means 27 at a vertical surface of a building or the like.

The belt 28 has a very specific shape. It comprises a number of outwardly extending protrusions29, wherein the belt 28 is concavely deformed between the protrusions 29. This can be see in more detail in figure 8.

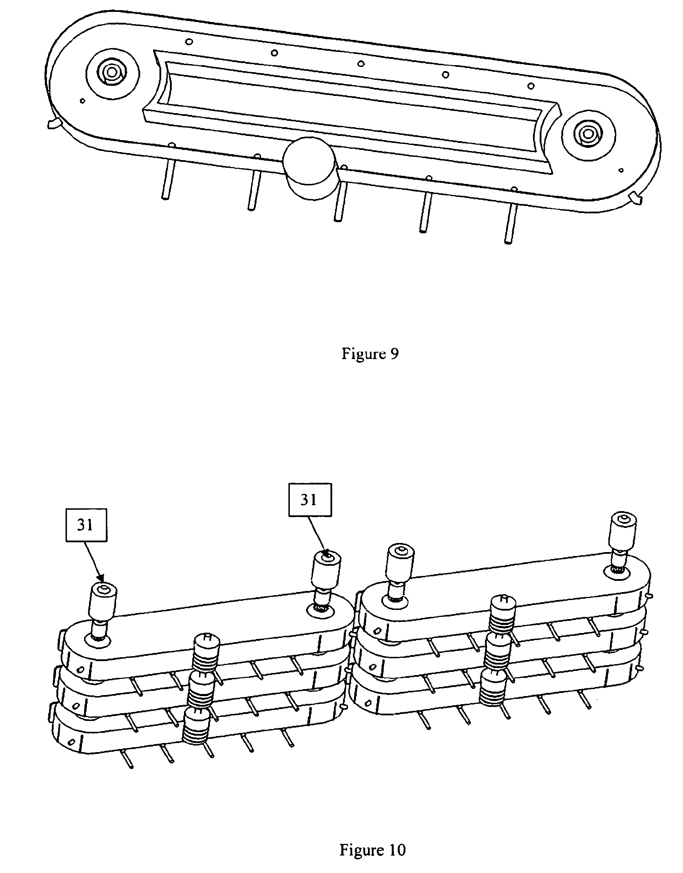

Figure 9 illustrates that the container 21 additionally comprises an empty chamber 30 inside thereof for algae which do not grow densely on the backside of the unit.

Finally, figure 10 illustrates a modular design of a photo-bioreactor according to the invention which can be driven by a belt motor 31. Again, the modular container 21 can be operated in serious or parallel.

In operation, the container 21 of the invention can be filled with algae culture and supplied with gases and liquids as explained above for the reactors of figures 1-6. Also discharge of gas can be achieved via a hole (not shown) on the top of the container 21.

The photo-bioreactor of the invention is especially suitable for kinds of algae that require a surface to grow over, such as ulva (sea lettuce). It can be also used to grow some kinds of sea weed.

The belt 28 allows certain kinds of algae to stick to and to grow thereon. The protrusions 29 of the belt 28, when rotated, clean the transparent inner surface of the container 21, preferably by scraping, and move the algae periodically to allow for equal distribution of the liquid feeding substances. The screw 25 can be used to harvest algae from a cavity formed by the concave deformation of the belt limited by two protrusions and the associated part of the inner surface of the container 21. For harvesting, the screw 25 is lowered into the respective concavity, and the screw rotates to scoop up the algae that is on the concave surface of the belt 28 and/or on the inner surface of the container 21, and then the screw 25 is raised up. Thereafter, the belt 28 moves to center the next cavity under the screw 25 which is then lowered again to scoop up the algae. The screw 5 thus more or less functions as a brush or scrubber.

## Claims

1. Photo-bioreactor for growing algae comprising:
- at least one transparent container (21) having an at least partly round perimeter and containing algae culture,
- optional means for mounting the container (27) on a vertical surface, preferably of a glass walled building,
- at least one feed gas tube (24),
- at least one gas removal hole,
- at least one feed liquid tube (22),
- at least one liquid discharge tube (26), preferably provided on the perimeter of the container, and
- rotatable stirring and removal means for stirring the algae culture and removing algae formed inside the container, wherein the stirring and removal means comprises a rotatable belt (28) having protrusions extending outwardly, and the belt being concavely deformed between the protrusions, and the stirring and removal means comprises a screw (25) adapted to be lowered into a concave deformation of the belt and to be raised there from.

2. Photo-bioreactor according to claim 1, wherein the protrusions of the belt, when rotated, clean the transparent inner surface of the container.

3. Photo-bioreactor according to any of the preceding claims, comprising at least one drive mechanism for rotating the stirring and removal means.

4. Photo-bioreactor according to any of the preceding claims, wherein the stirring and removal means comprises a bearing which is located above the level of liquids in the container.

5. Photo-bioreactor according to any of the preceding claims, wherein the stirring and removal means comprises a rotating shaft isolated from any contact with the liquids in the container.

6. Photo-bioreactor according to claim 1, wherein the container has an elongated shape with rounded corners.

7. Building, preferably an at least partly glass walled building, having at least one photo-bioreactor according to any of the preceding claims mounted on a vertical surface thereof.

8. Building according to claim 7, wherein the at least one photo-bioreactor is connected with the air conditioning system of the building, preferably the at least one feed gas tube.

## Patentansprüche

1. Photobioreaktor zur Algenzucht, welcher umfasst:
- wenigstens einen transparenten Behälter (21) mit einem wenigstens teilweise runden Umfang und enthaltend Algenkultur,
- optional Mittel zum Montieren des Behälters (27) auf einer vertikalen Oberfläche, bevorzugt eines glaswandigen Gebäudes,
- wenigstens eine Beschickungsgasleitung (24),
- wenigstens ein Gasentfernungsloch,
- wenigstens eine Beschickungsflüssigkeitsleitung (22),
- wenigstens eine Flüssigkeitsabgabeleitung (26), die bevorzugt am Umfang des Behälters bereitgestellt ist, und
- ein drehbares Rühr- und Entfernungsmittel zum Rühren der Algenkultur und zum Entfernen von innerhalb des Behälters gebildeten Algen, wobei das Rühr- und Entfernungsmittel einen drehbaren Gurt (28) mit sich nach außen erstreckenden Vorsprüngen umfasst, und wobei der Gurt konkav zwischen den Vorsprüngen deformiert ist, und wobei das Rühr- und Entfernungsmittel eine Schnecke (25) umfasst, die angepasst ist, um in eine konkave Deformation des Gurts abgesenkt und aus dieser angehoben zu werden.

2. Photobioreaktor nach Anspruch 1, wobei die Vorsprünge des Gurts, wenn er gedreht wird, die transparente innere Oberfläche des Behälters reinigen.

3. Photobioreaktor nach einem der vorangehenden Ansprüche, umfassend wenigstens einen Antriebsmechanismus zum Drehen des Rühr- und Entfernungsmittels.

4. Photobioreaktor nach einem der vorangehenden Ansprüche, wobei das Rühr- und Entfernungsmittel ein Lager umfasst, das oberhalb des Niveaus der Flüssigkeiten in dem Behälter angeordnet ist.

5. Photobioreaktor nach einem der vorangehenden Ansprüche, wobei das Rühr- und Entfernungsmittel eine Drehwelle umfasst, die von jeglichem Kontakt mit den Flüssigkeiten in dem Behälter isoliert ist.

6. Photobioreaktor nach Anspruch 1, wobei der Behälter eine längliche Form mit abgerundeten Ecken aufweist.

7. Gebäude, bevorzugt ein wenigstens teilweise glaswandiges Gebäude, mit wenigstens einem Photobioreaktor gemäß einem der vorangehenden Ansprüche, der an einer vertikalen Oberfläche desselben montiert ist.

8. Gebäude nach Anspruch 7, wobei der wenigstens eine Photobioreaktor mit dem Belüftungssystem des Gebäudes, bevorzugt der wenigstens einen Beschickungsgasleitung, verbunden ist.

## Revendications

1. Photo-bioréacteur pour faire croître des algues comprenant :
- au moins un récipient transparent (21) ayant un périmètre au moins partiellement arrondi et contenant une culture d'algues,
- un moyen facultatif pour monter le récipient (27) sur une surface verticale, de préférence d'un bâtiment vitré,
- au moins un tube de gaz d'alimentation (24),
- au moins un trou d'évacuation de gaz,
- au moins un tube de liquide d'alimentation (22),
- au moins un tube de décharge de liquide (26), prévu de préférence sur le périmètre du récipient, et
- un moyen de retrait et d'agitation rotatif pour agiter la culture d'algues et enlever les algues formées à l'intérieur du récipient, dans lequel le moyen de retrait et d'agitation comprend une ceinture rotative (28) ayant des protubérances s'étendant vers l'extérieur, et la ceinture étant déformée de manière concave entre les protubérances, et le moyen de retrait et d'agitation comporte une vis (25) conçue pour être abaissée dans une déformation concave de la ceinture et pour en être soulevée.

2. Photo-bioréacteur selon la revendication 1, dans lequel les protubérances de la ceinture, lorsqu'elle est en rotation, nettoient la surface intérieure transparente du récipient.

3. Photo-bioréacteur selon l'une quelconque des revendications précédentes, comprenant au moins un mécanisme d'entraînement pour mettre le moyen de retrait et d'agitation en rotation.

4. Photo-bioréacteur selon l'une quelconque des revendications précédentes, dans lequel le moyen de retrait et d'agitation comprend un roulement qui se trouve au-dessus du niveau des liquides dans le récipient.

5. Photo-bioréacteur selon l'une quelconque des revendications précédentes, dans lequel le moyen de retrait et d'agitation comprend un arbre rotatif n'ayant aucun contact avec les liquides dans le récipient.

6. Photo-bioréacteur selon la revendication 1, dans lequel le récipient a une forme allongée avec des coins arrondis.

7. Bâtiment, de préférence un bâtiment au moins partiellement vitré, ayant au moins un photo-bioréacteur selon l'une quelconque des revendications précédentes monté sur l'un de ses murs.

8. Bâtiment selon la revendication 7, dans lequel l'au moins un photo-bioréacteur est lié au système de climatisation du bâtiment, de préférence au moins au tube de gaz d'alimentation.
